# EUROPÄISCHE PATENTANMELDUNG

(11) **EP 1 304 070 A2**
(43) Veröffentlichungstag der Anmeldung: **23.04.2003**
(21) Anmeldenummer: 02022529.8
(22) Anmeldetag: 07.10.2002
(51) Int. Cl.: A61B 5/00, A61B 6/03

(54) **Bildgebungsverfahren und Bildgebungsvorrichtung, insbesondere für die Kleintierbildgebung**

(30) Priorität: 19.10.2001 DE 10151670
(71) Anmelder: SIEMENS AKTIENGESELLSCHAFT, 80333 München (DE)
(72) Erfinder: Mertelmeier, Thomas, Dr., 91058 Erlangen (DE)

(57) **Zusammenfassung**

Die Erfindung betrifft ein Bildgebungsverfahren und eine Bildgebungsvorrichtung, insbesondere für die Kleintierbildgebung, bei dem das zu untersuchende Objekt (15) mit einem aktivierbaren optischen Kontrastmittel, das vorzugsweise eine metabolisch aktivierbare Markierung aufweist, versetzt und von einer ersten optischen Anregungsquelle (7) bestrahlt wird, wobei die vom Objekt (15) reflektierte erste Strahlung (13) von einem ersten optischen Detektor (9) erfasst wird, und wobei das zu untersuchende Objekt (15) gleichzeitig von einer zweiten tomographischen Anregungsquelle (1) bestrahlt wird, während die vom Objekt (15) transferierte zweite Strahlung (5) von einem zweiten tomographischen Detektor (2) erfasst wird. Die zweite tomographische Anregungsquelle (1) erzeugt dabei mit Vorteil eine Röntgenstrahlung, so dass das resultierende CT-Bild mit dem optischen Bild zur Auswertung morphologischer und funktionaler Informationen überlagert werden kann.

## Beschreibung

Die Erfindung betrifft ein Bildgebungsverfahren nach dem Oberbegriff des Patentanspruches 1 sowie eine Bildgebungsvorrichtung nach dem Oberbegriff des Patentanspruches 9, insb. für die Kleintierbildgebung.

Bekannte Bildgebungsverfahren und Vorrichtungen für die Kleintierbildgebung bestehen aus einer ersten optischen Anregungsquelle, die ein mit einem aktivierbaren optischen Kontrastmittel versetztes Objekt, wie beispielsweise eine Maus oder eine Ratte, bestrahlt, während die vom Objekt reflektierte Strahlung von einem optischen Detektor erfasst wird. Bei Untersuchungen von Stoffwechselfunktionen am lebenden Kleintier werden aktivierbare optische Kontrastmittel verwendet, die insb. im nahen Infrarotbereich fluoreszieren. Das Kontrastmittel ist im gesunden Gewebe inert und wird erst im zu detektierenden Zielgewebe, beispielsweise einen Tumor, durch krankheitskorrelierte Stoffwechselaktivitäten (enzymatische Prozesse) aktiviert, d.h. in einen fluoreszenten Zustand überführt. Durch einen hochselektiven Aktivierungsmechanismus wird mit diesen Kontrastmitteln ein sehr hohes Signal-Rausch-Verhältnis erreicht. Hierzu weisen die Kontrastmittel metabolische Markierungen auf, die auf bestimmte Stoffwechselfunktionen reagieren und das Kontrastmittel aktivieren. Dadurch sind im wesentlichen funktionale Informationen des Zielgebiets erfassbar.

Derartige Bildgebungsverfahren und Vorrichtungen sind beispielsweise aus den DE 195 23 806 A1 und DE 198 04 797 A1 bekannt. Diese zeigen ein Beleuchtungssystem mit einer optischen Lichtquelle, die Anregungslicht emittiert, das dem Fluoreszenzanregungsspektrum des zu untersuchenden Gewebes angepasst ist. Die Intensitäten der reflektierten Strahlung werden von einem optischen Detektor erfasst und ausgewertet. Dieser erfasst auch die Fluoreszenzstrahlung der interessierenden Bereiche. DE 195 23 806 A1 offenbart darüber hinaus eine zweite optische Lichtquelle, die teilweise im Strahlengang der ersten Lichtstrahlung auf die fluoreszierende Stoffe aufweisende Oberfläche verläuft und ein für den Betrachter stehendes Bild der Verteilung fluoreszierender Stoffe auf der Oberfläche erzeugt, falls der erste Lichtstrahl eine ausreichend schnelle Scanbewegung der Oberfläche vollzieht. Auch die DE 198 04 797 A1 offenbart die Verwendung einer zweiten optischen Lichtquelle, die das Objektfeld bzw. die Oberfläche zur visuellen Beobachtung beleuchtet.

Diese optische Bildgebungsmodalität hat jedoch den Nachteil, dass aufgrund der starken Streuung und der Absorption von Licht im Zielgewebe die Ortsauflösung der reflektierten Strahlung stark beschränkt ist. Es lassen sich somit kaum anatomische und/oder morphologische Informationen des Orts der Untersuchung erfassen.

Eine andere Bildgebungsmodalität ist die Mikro-CT (Computer-Tomographie). Diese liefert anatomische und/oder morphologische Informationen mit hoher Ortsauflösung, da entsprechende Röntgenstrahlung durch das zu untersuchende Gewebe absorbiert wird und die transferierte Strahlung dadurch mit hoher Genauigkeit anatomische Verhältnisse widerspiegelt. Andererseits ist die Mikro-CT wegen der relativ geringen Röntgenabsorption unsensitiv gegenüber stoffwechselspezifischen Kontrastmitteln, die beispielsweise für die Nuklearmedizin verwendet werden.

Es sind weiterhin Bildgebungsverfahren bekannt, wonach zunächst mittels einer Röntgenaufnahme anatomische und morphologische Informationen ermittelt werden, um dann mit Hilfe optischer Bildgebungsverfahren funktionale Schnittbilder zu ermitteln, die dann mit Hilfe der Röntgenbilder ausgewertet werden. Diese Verfahren sind jedoch mit dem Nachteil behaftet, dass in aller Regel keine eineindeutige Zuordnung der funktionalen Informationen zu den anatomischen Informationen möglich ist und eine genaue Auswertung der Bildinformationen daher nur mit Hilfe entsprechender Erfahrung möglich war.

Der vorliegenden Erfindung liegt daher die Aufgabe zugrunde, ein Bildgebungsverfahren bzw. eine Bildgebungsvorrichtung herkömmlicher Bauart derart zu verbessern, dass sowohl anatomische Informationen mit hoher Ortsauflösung als auch funktionale Informationen mit hoher Empfindlichkeit von einem Zielgewebe erfasst werden können.

Zur Lösung dieser Aufgabe dienen die kennzeichnenden Merkmale der unabhängigen Patentansprüche 1 und 9. Vorteilhafte Ausgestaltungen der vorliegenden Erfindung sind in den Unteransprüchen gekennzeichnet und beansprucht.

Erfindungsgemäß wird bei einem insb. für die Kleintierbildgebung geeignetes Bildgebungsverfahren das zu untersuchende Objekt mit einem aktivierbaren optischen Kontrastmittel versetzt und von einer ersten optischen Anregungsquelle bestrahlt, wobei die vom Objekt reflektierte erste Strahlung von einem ersten optischen Detektor erfasst wird. Darüber hinaus wird das zu untersuchende Objekt gleichzeitig von einer zweiten Anregungsquelle bestrahlt, wobei die vom Objekt transferierte zweite Strahlung der zweiten Anregungsquelle von einem zweiten Detektor erfasst wird. Mit Vorteil ist somit das optische Bildgebungssystem mit dem tomographischen Bildgebungssystem kombiniert, ohne dass das Objekt verschoben werden muss. Es werden somit gleichzeitig verschiedene Informationen desselben Zielgewebes ermittelt, die nicht nur jeweils einzeln ausgewertet werden, sondern die aufgrund der Tatsache, dass beide Bildgebungsverfahren gleichzeitig durchgeführt werden, auch untereinander korreliert werden können.

Mit Vorteil wird das zu untersuchende Objekt zunächst mit einem optischen Fluoreszenzkontrastmittel versetzt, das mindestens eine metabolisch aktivierbare Markierung aufweist, so dass mittels des ersten Detektors zurückgestrahlte Fluoreszenzstrahlung erfassbar ist. Die erfasste zurückgestrahlte Fluoreszenzstrahlung wird rekonstruiert, so dass ein entsprechendes Bild mit funktionalen Informationen entsteht. Die Rekonstruktion für die optische Tomographie wird dabei mit Vorteil iterativ durchgeführt (z.B. R. Gaudette et al: Phys. Med. Biol. 45, 1051-1070 (2000), A.D. Klose, A.H. Hielscher: Med. Phys. 26, 1698-1707 (1999), H. Dehghani, D.T. Delpy, S.R. Arridge: Phys. Med. Biol. 44, 2897-2906 (1999), S.A. Arridge, J.C. Hebden, Phys. Med. Biol. 42, 841-853 (1997)).

Die zweite Anregungsquelle ist mit Vorteil eine Röntgenröhre, die eine Röntgenstrahlung erzeugt. Diese Röntgenstrahlung durchleuchtet das Objekt und wird von dem beispielsweise als CT-Detektor ausgebildeten zweiten Detektor detektiert. Das dadurch ermittelte Röntgenbild enthält entsprechende anatomische und morphologische Informationen mit hoher Ortsauflösung. Die zweite Anregungsquelle könnte aber auch ein Ultraschallwandler oder ein Kernspintomograph sein.
Die durch die Röntgenaufnahme messbaren Röntgenschwächungskoeffizienten können mit Vorteil als Vorabinformationen verwendet werden. Mit Vorteil lässt sich mittels des Schwächungskoeffizienten der vom Objekt transferierten zweiten Röntgenstrahlung die Ausgangskonzentration des Kontrastmittels bestimmen, die beispielsweise zur Quantifizierung der Stoffwechselaktivität mittels einer Bestimmung der Aktivierungsrate herangezogen werden kann.

Weiterhin dient beispielsweise der Röntgenschwächungskoeffizient mit Vorteil zur Bestimmung optischer Streu- und/oder Absorptionskoeffizienten für die Auswertung der ersten reflektierten optischen Strahlung. Vorzugsweise handelt es sich bei der ersten optischen Bildgebung um Fluoreszenz im nahen Infrarotbereich (NIRF), wofür neue intelligente Fluoreszenzkontrastmittel entwickelt wurden (vgl. R. Weissleder et al.: Nature Biotechnology 17, 375-368 (1999)). Somit lässt sich von jedem mittels des optischen Bildgebungsverfahrens ermittelten Voxel ein Absorptions- und Streukoeffizient schätzen. Die Fluoreszenzaktivität kann daher qualitativ und quantitativ exakter bestimmt werden.

Mit Vorteil wird die vom ersten Detektor erfasste reflektierte erste Strahlung ausgewertet und in entsprechende funktionale Bildinformationen umgewandelt. Die vom zweiten Detektor erfasste transferierte zweite Strahlung wird ebenfalls ausgewertet, so dass ein Bilddatensatz mit morphologischen Bildinformationen entsteht. Die daraus resultierenden einzelnen Bildinformationen können dann zu einem Gesamtbild mit morphologischen und funktionalen Informationen desselben Zielgewebes überlagert werden.

Erfindungsgemäß lassen sich auch mehrere Schnittbilder des Objekts zu einem dreidimensionalen Bilddatensatz ergänzen. Hierfür werden die vom zweiten Detektor erfassten transferierten zweiten Strahlungen, die mehrere Schnitte des Objekts darstellen, ausgewertet und zur Erzeugung des dreidimensionalen Bilddatensatzes herangezogen. Fehlende Bilddaten zwischen den einzelnen zweidimensionalen Schnittbildern werden nach bekannten Verfahren interpoliert oder geschätzt (Volume rendering). Im Anschluss daran können die dreidimensionalen Bildinformationen mittels der funktionalen Bildinformationen des ersten Detektors ergänzt bzw. überlagert werden, um einen dreidimensionalen Bilddatensatz zu erhalten, der auch funktionale Bildinformationen enthält. Zur genauen Zuordnung der verschiedenen Bildinformationen und zur Vermeidung von Lageartefakten können anatomische und/oder künstliche Landmarken verwendet werden. Hierzu dienen beispielsweise kleine Leuchtdioden auf der Oberfläche eines Objektträgers oder entsprechende Metallkügelchen, die im CT-Bild sichtbar sind.

Auch lassen sich die optischen Funktionsinformationen aus einem planaren optischen Bild in das Röntgenprojektionsbild überlagern, das mit dem gleichen Projektionswinkel aufgenommen wurde.

Die Erfindung verfügt daher über verschiedene Vorteile gegenüber den herkömmlichen Systemen. Es lassen sich sowohl anatomische als auch funktionale Informationen mit einem Gerät detektieren. Das System kann dezentral aufgestellt werden und verfügt über kleine Abmessungen. Weiterhin ist es kostengünstiger als andere "Single Modality"-Systeme, wie beispielsweise PET oder MRI. Die optische Rekonstruktion lässt sich mittels der CT-Informationen zuverlässig verbessern. Anatomische Informationen mit hoher Ortsauflösung und funktionale Informationen mit hoher Empfindlichkeit werden dadurch gleichzeitig bereitgestellt.

Eine bevorzugte Ausführungsform der vorliegenden Erfindung wird anhand der Zeichnungen näher erläutert. Dabei zeigen:
- Figur 1: eine schematische Darstellung der erfindungsgemäßen Bildgebungsvorrichtung,
- Figur 2: eine schematische Darstellung einer alternativen Bildgebungsvorrichtung, und
- Figur 3: eine zweite alternative Ausführungsform der erfindungsgemäßen Bildgebungsvorrichtung.

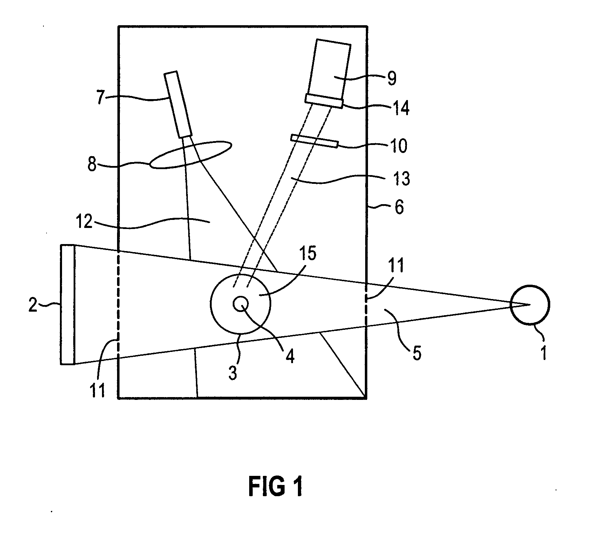

Figur 1 zeigt schematisch eine erfindungsgemäße Bildgebungsvorrichtung mit einer ersten optischen Anregungsquelle 7, die ein zu untersuchendes Objekt 15, das mit einem aktivierbaren optischen Kontrastmittel versetzt wurde, bestrahlt und einen ersten optischen Detektor 9, der vom Objekt 15 reflektierte erste Strahlung 13 erfasst. Gleichzeitig bestrahlt eine zweite Anregungsquelle 1 das zu untersuchende Objekt 15. Ein zweiter Detektor 2 erfasst die vom Objekt 15 transferierte zweite Strahlung 5.

Mit Vorteil ist die zweite Anregungsquelle 1 eine Röntgenröhre und der zweite Detektor 2 ein Röntgen-Detektor. Ein Objektträger 3, der das Objekt 15 hält, ist mit Vorteil ein Zylinder aus Glas oder Plexiglas, der um eine Drehachse 4 schwenk- bzw. drehbar ist. Dieser Objektträger 3 ist sowohl für Röntgenstrahlung als auch für Licht durchlässig. Während das Objekt 15 auf dem Objektträger 3 drehbar (Drehteller) gelagert ist, ist sowohl das Röntgensystem (1, 2) als auch das optische Aufnahmesystem (7, 9) ortsfest angeordnet. Mit Vorteil findet sich das optische Aufnahmesystem innerhalb eines Gehäuses 6, das lichtundurchlässig, d.h. lichtdicht ist.

Die zweite Strahlung 5, d.h. beispielsweise der Röntgenkegelstrahl, der von der Röntgenröhre 1 abgestrahlt wird, durchdringt ein röntgenstransparentes Fenster 11 des lichtdichten Gehäuses 6 auf beiden Seiten des Objekts 15 und wird von dem zweiten Detektor 2 detektiert.

Die erste Anregungsquelle 7 ist mit Vorteil eine Infrarot-Laserdiode, die infrarotes Licht über eine Linse 8 auf das Objekt 15 strahlt. Die erste einfallende Strahlung 12, beispielsweise Infrarotlicht, regt das im Objekt vorhandene optische Kontrastmittel an, wodurch eine reflektierte erste Strahlung 13 entsteht, d.h. beispielsweise reflektiertes Fluoreszenzlicht. Diese reflektierte Strahlung 13 wird über einen Filter 10 und eine Linse 14 von dem ersten Detektor 9, beispielsweise einer CCD-Kamera detektiert.

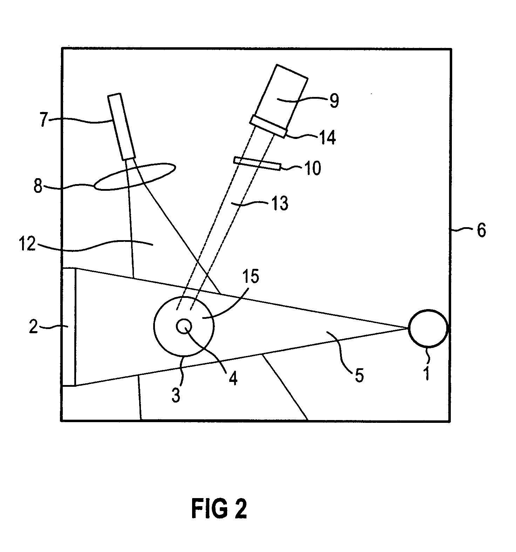

Figur 2 zeigt eine alternative Ausführungsform der erfindungsgemäßen Bildgebungsvorrichtung. Diese unterscheidet sich von der Ausführungsform nach Figur 1 dadurch, dass sich auch die zweite Anregungsquelle 1 und der zweite Detektor 2 innerhalb des lichtdichten Gehäuses 6 befindet.

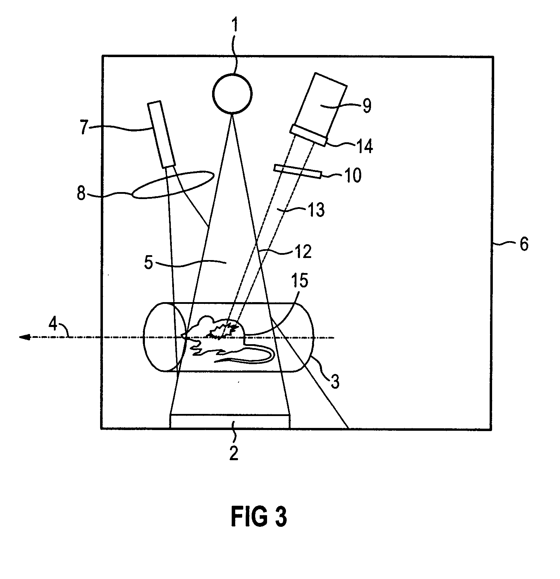

Figur 3 zeigt eine weitere alternative Ausführungsform der erfindungsgemäßen Bildgebungsvorrichtung. Auch hier befinden sich beide bildgebenden Systeme innerhalb des lichtdichten Gehäuses 6, während das Objekt 15 sukzessive entlang der Achse 4 verschoben wird. Hierdurch lassen sich sukzessive verschiedene Schnittbilder des zu untersuchenden Gewebes aufnehmen, die dann zu einem dreidimensionalen CT-Bild zusammengesetzt werden können. Bei Verwendung eines flächenhaften Röntgendetektors 2 kann das ganze Objekt 3-D-rekonstruiert werden (Kegelstrahltomographie). Dieses dreidimensionale Bild lässt sich mittels der ebenfalls gleichzeitig erhaltenen optischen Bildern mit den funktionalen Informationen ergänzen.

Der rekonstruierte Röntgenschwächungskoeffizient an einem Ort des Zielgewebes wird mit Vorteil als Schätzwert für den optischen Absorptions- und/oder Streukoeffizienten an diesem Ort bei der iterativen optischen Rekonstruktion verwendet, so dass sich das inverse Problem der optischen Bildgebung entschärfen lässt.

## Patentansprüche

1. Bildgebungsverfahren, insbesondere für die Kleintierbildgebung, bei dem das zu untersuchende Objekt (15) mit einem aktivierbaren optischen Kontrastmittel versetzt und von einer ersten optischen Anregungsquelle (7) bestrahlt wird, und wobei die vom Objekt (15) reflektierte erste Strahlung (13) von einem ersten optischen Detektor (9) erfasst wird,
**dadurch gekennzeichnet,**
**dass** das zu untersuchende Objekt (15) gleichzeitig von einer zweiten tomographischen Anregungsquelle (1) bestrahlt wird, und dass die vom Objekt (15) transferierte zweite Strahlung (5) von einem zweiten tomographischen Detektor (2) erfasst wird.

2. Bildgebungsverfahren nach Anspruch 1,
**dadurch gekennzeichnet,**
**dass** das zu untersuchende Objekt (15) mit einem optischen Fluoreszenzkontrastmittel versetzt wird, das mindestens eine metabolisch aktivierbare Markierung aufweist, so dass mittels des ersten Detektors (9) zurückgestrahlte Fluoreszenzstrahlung erfasst wird.

3. Bildgebungsverfahren nach einem der Ansprüche 1 oder 2,
**dadurch gekennzeichnet,**
**dass** die zweite tomographische Anregungsquelle (1) eine Röntgenstrahlung erzeugt, und dass der zweite tomographische Detektor (2) ein CT-Detektor ist.

4. Bildgebungsverfahren nach einem der vorhergehenden Ansprüche,
**dadurch gekennzeichnet,**
**dass** ein Schwächungskoeffizient der vom Objekt (15) transferierten zweiten Strahlung (5) verwendet wird, um die Ausgangskonzentration des Kontrastmittels zu bestimmen.

5. Bildgebungsverfahren nach einem der vorhergehenden Ansprüche,
**dadurch gekennzeichnet,**
**dass** ein Schwächungskoeffizient der vom Objekt (15) transferierten zweiten Strahlung (5) verwendet wird, um optische Streu- und/oder Absorptionskoeffizienten für die Auswertung der ersten Strahlung (12) zu bestimmen.

6. Bildgebungsverfahren nach einem der vorhergehenden Ansprüche,
**dadurch gekennzeichnet,**
**dass** die vom ersten optischen Detektor (9) erfasste reflektierte erste Strahlung (13) ausgewertet und in funktionale Bildinformationen umgewandelt wird,
**dass** die vom zweiten tomographischen Detektor (2) erfasste transferierte zweite Strahlung (5) ausgewertet und in morphologische Bildinformationen umgewandelt wird,
und **dass** die daraus resultierenden einzelnen Bildinformationen zu einem Gesamtbild mit morphologischen und funktionalen Informationen überlagert werden.

7. Bildgebungsverfahren nach einem der vorhergehenden Ansprüche,
**dadurch gekennzeichnet,**
**dass** vom zweiten tomographischen Detektor (2) erfasste transferierte zweite Strahlungen (5), die mehrere Schnitte des Objekts (15) darstellen, ausgewertet und zur Erzeugung eines dreidimensionalen Bilddatensatzes herangezogen werden, und
**dass** die funktionalen Bildinformationen des ersten optischen Detektors (9) mit dem dreidimensionalen Bilddatensatz überlagert werden.

8. Bildgebungsverfahren nach einem der vorhergehenden Ansprüche 6 oder 7,
**dadurch gekennzeichnet**
**dass** zur Überlagerung der Informationen anatomische und/oder künstliche Landmarken verwendet werden.

9. Bildgebungsvorrichtung, insbesondere für die Kleintierbildgebung, mit
einer ersten optischen Anregungsquelle (7), die ein zu untersuchendes Objekt (15), das mit einem aktivierbaren optischen Kontrastmittel versetzt wurde, bestrahlt, und
einem ersten optischen Detektor (9), der vom Objekt (15) reflektierte erste Strahlung (13) erfasst,
**dadurch gekennzeichnet,**
**dass** gleichzeitig eine zweite tomographische Anregungsquelle (1) das zu untersuchende Objekt (15) bestrahlt, und
**dass** ein zweiter tomographischer Detektor (2) die vom Objekt (15) transferierte zweite Strählung (5) erfasst.

10. Vorrichtung nach Anspruch 9,
**dadurch gekennzeichnet,**
**dass** die zweite tomographische Anregungsquelle (1) eine Röntgenröhre ist, und dass der zweite tomographische Detektor (2) ein CT-Detektor ist.

11. Vorrichtung nach einem der Ansprüche 9 oder 10,
**dadurch gekennzeichnet,**
**dass** die erste optische Anregungsquelle (7) eine Infrarot-Laserquelle ist, und dass der erste Detektor (9) eine CCD-Kamera ist.

12. Vorrichtung nach einem der Ansprüche 9 - 11,
**dadurch gekennzeichnet,**
**dass** das Objekt (15) auf einem insbesondere um eine Drehachse (4) drehbaren Objektträger (3) aus Glas oder Plexiglas montierbar ist.
